# EUROPEAN PATENT SPECIFICATION

(11) **EP 4 454 598 B1**
(45) Date of publication and mention of the grant of the patent: **09.09.2026**
(21) Application number: 24172521.7
(22) Date of filing: 25.04.2024
(51) Int. Cl.: A61C 1/05, A61B 17/32, A61B 17/3207, B21D 39/04, F16L 13/00

(54) **MEDICAL CUTTING DEVICE AND CARTRIDGE**
MEDIZINISCHE SCHNEIDVORRICHTUNG UND KARTUSCHE
DISPOSITIF DE COUPE MÉDICAL ET CARTOUCHE

(30) Priority: 27.04.2023 JP 2023073845
(43) Date of publication of application: 30.10.2024
(73) Proprietor: Nakanishi Inc., Kanuma-shi, Tochigi 322-8666 (JP)
(72) Inventor: INOUE, Yamato, Tochigi, 322-8666 (JP)
(74) Representative: Lewis Silkin LLP

(56) References cited:
- CN-U- 208 274 634
- JP-A- 2020 174 901
- US-A- 5 254 004
- US-A1- 2002 124 443

## Description

### TECHNICAL FIELD

The present invention relates to a medical cutting device used for dental or surgical treatment, and a cartridge detachably attached to the medical cutting device.

### BACKGROUND ART

A medical cutting device such as an air turbine handpiece is known in the related art. For example, in dental treatment, various medical cutting devices are used, such as an air turbine handpiece and a micromotor handpiece each including a head attached with a cutting tool such as a diamond point bar, a carbide bar, a file, or a reamer. For example, JP2020-174901A discloses an air turbine handpiece that is a medical cutting device.

In the air turbine handpiece disclosed in JP2020-174901A, a turbine rotor and a spindle are accommodated in a head portion. The spindle has a hollow and substantially cylindrical shape, and is rotatably supported by the head portion via a bearing. The turbine rotor is fixed to an outer peripheral surface of the spindle, and a cutting tool is inserted into a hollow interior of the spindle.

In this type of air turbine handpiece, the turbine rotor has been generally formed of aluminum in the related art from a viewpoint of ease of processing during molding.

On the other hand, in a dental air turbine disclosed in JP2005-000311A, a rotation portion (turbine rotor) is formed of stainless steel.

CN 208 274 634 U discloses a turbine dental drill handpiece having a movement assembly, a machine head, an intake duct, a cooling tube, a handle and a socket. The movement assembly is rotatably mounted in the head, and the top end of the head is further mounted with a back cover assembly. The movement assembly (2) includes a wind wheel (12) and a needle shaft. The wind wheel is fixedly sleeved outside the needle taking shaft, and the needle shaft is rotatably installed in the inner cavity of the head.

### SUMMARY OF INVENTION

However, since such a turbine rotor formed of stainless steel has a large inertial moment, there is a problem that a large load is applied to a joint portion that fixes a turbine rotor to a spindle when a cutting load is input. This may cause a failure due to heat generation or poor attachment and detachment of a cutting tool.

In addition, the turbine rotor using a material having a large inertial moment has a disadvantage of long inertial rotation until rotation stops when supply of high-pressure air is stopped.

The present invention provides a cartridge capable of increasing a fixing force of a joint portion that fixes a turbine rotor using a material having a large inertial moment such as stainless steel to a spindle and preventing the turbine rotor from falling off the spindle, and a medical cutting device to which the cartridge is detachably attachable.

According to the present invention, there are provided a cartridge detachably attached to a medical cutting device and a medical cutting device as defined in the appended claims.

### BRIEF DESCRIPTION OF DRAWINGS

Fig. 1 is a cross-sectional view of a head portion of an air turbine handpiece including a rotation portion according to an embodiment of the present invention.
Fig. 2 is a schematic view (a cross section of a turbine rotor and a side surface of a spindle) of the rotation portion.
Fig. 3A is a cross-sectional view of the turbine rotor.
Fig. 3B is a perspective view of the turbine rotor.
Fig. 4A is a side view of the spindle.
Fig. 4B is a cross-sectional view of the spindle.
Fig. 5A is a schematic view in which grooves at a joint portion of the rotation portion intersect with each other.
Fig. 5B is an enlarged view of the vicinity of the grooves shown in Fig. 5A.
Fig. 6 is a schematic view showing a cartridge accommodated in the head portion of the air turbine handpiece.

### DESCRIPTION OF EMBODIMENTS

Hereinafter, embodiments of a medical cutting device and an air turbine handpiece as an example of a cartridge detachably attached to the medical cutting device according to the present invention will be described with reference to the accompanying drawings. The drawings are viewed in orientations of reference numerals.

### First Embodiment

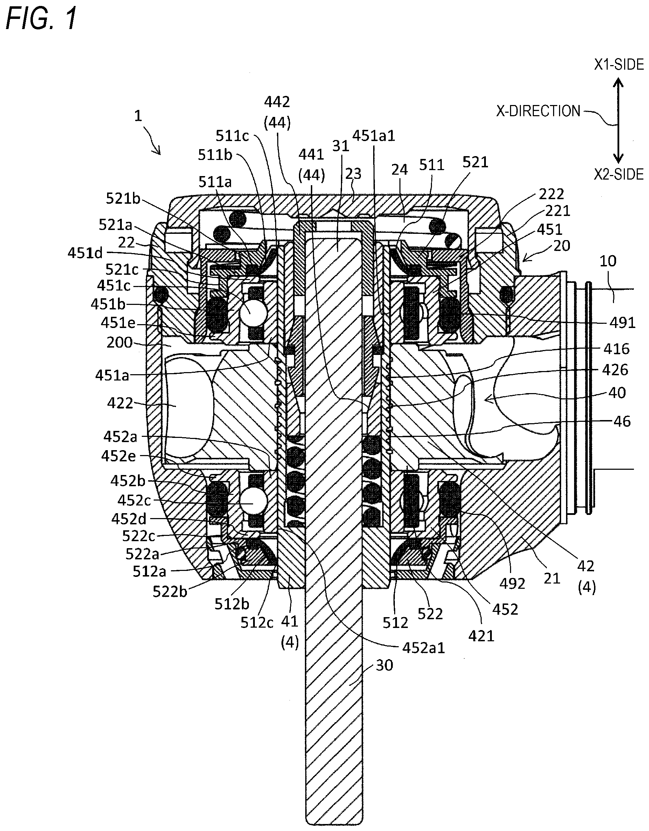

An air turbine handpiece 1 according to a first embodiment of the present invention will be described with reference to Figs. 1 to 5B.

As shown in Fig. 1, the air turbine handpiece 1 according to the present embodiment includes a handpiece main body 10 that is a main body of the air turbine handpiece 1, and a head portion 20 provided at a tip end portion of the handpiece main body 10. A cutting tool 30 is removably attached to the head portion 20. A plurality of types of cutting tools 30 may be attachable to the head portion 20 depending on a cutting object or the like to be cut using the air turbine handpiece 1.

A shaft portion 31 having a cylindrical shape is formed at one end portion of the cutting tool 30 attached to the head portion 20. The cutting tool 30 is attached to the head portion 20 by inserting the shaft portion 31 into the head portion 20. The one end portion of the cutting tool 30 (that is, a tip end portion of the shaft portion 31) is accommodated in the head portion 20, and the other end portion of the cutting tool 30 protrudes outward from the head portion 20.

Here, in order to simplify and clarify the description, an axial direction of the shaft portion 31 of the cutting tool 30 in the air turbine handpiece 1 in which the cutting tool 30 is attached to the head portion 20 is defined as an X-direction for convenience. The one end portion of the cutting tool 30 (that is, the tip end portion of the shaft portion 31) is defined as an X1-side, and the other end portion of the cutting tool 30 is defined as an X2-side. Unless otherwise specified, an axial direction, a peripheral direction, and a radial direction refer to directions based on the axial direction of the shaft portion 31.

Therefore, the shaft portion 31 is formed at an X1-side end portion of the cutting tool 30. The X1-side end portion of the cutting tool 30 is accommodated in the head portion 20, and an X2-side end portion thereof protrudes from the head portion 20 in an X2-direction.

The head portion 20 includes a main housing 21 having a substantially cylindrical shape with a bottom opened on the X1-side, and a head cap 22 that closes an opening, opened on the X1-side, of the main housing 21. An inner accommodation space 200 surrounded by the main housing 21 and the head cap 22 is formed in the head portion 20.

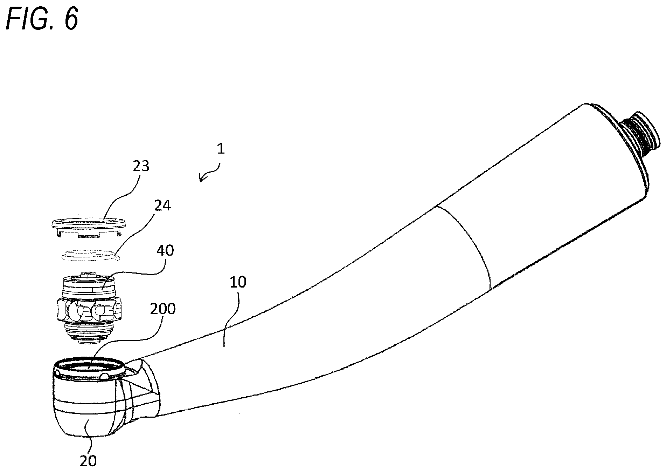

A cartridge 40, in which a spindle 41 having a hollow and substantially cylindrical shape extending in the X-direction, a turbine rotor 42 fixed to an outer peripheral surface of the spindle 41 by an adhesive, a chuck mechanism portion 44 supported on an inner peripheral surface of the spindle 41, a first bearing 451, a second bearing 452, and seal members 511 and 512 are modularized, is accommodated in the inner accommodation space 200 of the head portion 20. In the present embodiment, the cartridge 40 is attachable to and detachable from the head portion 20 (see Fig. 6). Therefore, the spindle 41, the turbine rotor 42, the chuck mechanism portion 44, the first bearing 451, the second bearing 452, and the seal members 511 and 512 can be replaced by replacing the cartridge 40 attached to the head portion 20. A plurality of types of cartridges 40 may be attachable to the head portion 20 depending on the cutting object or the like to be cut using the air turbine handpiece 1.

The head cap 22 includes a substantially annular outer member 221 fitted into and fixed to the main housing 21, and a substantially annular inner member 222 fixed to an inner peripheral surface of the outer member 221. The head cap 22 is provided on the X1-side with a push button 23 that is displaceable in the X-direction with respect to the main housing 21 and the head cap 22. The push button 23 is connected to the chuck mechanism portion 44 of the cartridge 40. A coil spring 24 is attached between the push button 23 and the head cap 22, and the push button 23 is biased to the X1-side with respect to the head cap 22 by the coil spring 24.

An X1-side end portion of the spindle 41 is rotatably supported by the first bearing 451. An X2-side end portion of the spindle 41 is rotatably supported by the second bearing 452. The first bearing 451 is fixed to an inner peripheral surface of the inner member 222 of the head cap 22 via an O-ring 491, and the second bearing 452 is fixed to an inner peripheral surface of the main housing 21 via an O-ring 492.

The turbine rotor 42 includes a substantially cylindrical rotation shaft portion 421 fitted to the outer peripheral surface of the spindle 41, and a plurality of turbine blades 422 extending radially outward with respect to the axial direction of the rotation shaft portion 421 and having blade shapes. The rotation shaft portion 421 and the turbine blades 422 may be integrally molded of the same material, or may be formed of different materials and integrated by joining. In the present embodiment, the rotation shaft portion 421 and the turbine blades 422 are integrally molded of the same material. When the turbine rotor 42 rotates, the spindle 41 also rotates integrally with the turbine rotor 42.

The chuck mechanism portion 44 has a hollow and substantially cylindrical shape, and includes a chuck portion 441 fitted to and supported on the inner peripheral surface of the spindle 41, and a pusher 442 that is provided at the X1-side end portion of the spindle 41 and operates the chuck portion 441. The cutting tool 30 is supported by the chuck portion 441 so as not to be displaced in the radial direction with respect to the spindle 41.

The pusher 442 is fitted to the inner peripheral surface of the spindle 41 and is slidably supported in the X-direction. The pusher 442 operates the chuck portion 441 to switch between a state in which an X1-side end portion of the shaft portion 31 of the cutting tool 30 is held by the chuck portion 441 and a state in which the X1-side end portion is not held by the chuck portion 441.

When the user presses the push button 23 toward the X2-side, the pusher 442 connected to the push button 23 slides toward the X2-side. The chuck portion 441 is switchable between an open state and a closed state, and is normally in the closed state. In the chuck mechanism portion 44, when the pusher 442 slides toward the X2-side due to the user pressing the push button 23 toward the X2-side, the chuck portion 441 is in the open state.

When attaching the cutting tool 30 to the head portion 20, the user presses the push button 23 toward the X2-side, whereby the pusher 442 slides toward the X2-side and the chuck portion 441 is maintained in the open state such that the cutting tool 30 can be received. When the chuck portion 441 is maintained in the open state, the shaft portion 31 of the cutting tool 30 is inserted into a hollow interior of the spindle 41 from the X2-side, and a tip end of the shaft portion 31 of the cutting tool 30 abuts on the chuck mechanism portion 44. When the user releases the push button 23 in a state in which the tip end of the shaft portion 31 of the cutting tool 30 abuts on the chuck mechanism portion 44, the pusher 442 slides toward the X1-side, the chuck portion 441 is in the closed state in which the shaft portion 31 of the cutting tool 30 is held by the chuck portion 441, and the cutting tool 30 is maintained in a state of being held by the chuck mechanism portion 44 and attached to the head portion 20 via the cartridge 40. Accordingly, the cutting tool 30 is rotatable integrally with the spindle 41 and the turbine rotor 42.

When detaching the cutting tool 30 from the head portion 20, the user presses the push button 23 toward the X2-side in the state in which the cutting tool 30 is held by the chuck portion 441, whereby the pusher 442 slides toward the X2-side and the chuck portion 441 is in the open state such that the cutting tool 30 can be detached. When the user pulls out the cutting tool 30 toward the X2-side while pressing the push button 23 toward the X2-side, the cutting tool 30 is removed from the chuck mechanism portion 44 and detached from the head portion 20.

The handpiece main body 10 includes a supply duct (not shown) that supplies high-pressure air (compressed air), which is an operating gas of the turbine rotor 42, to the head portion 20, and a discharge duct (not shown) that discharges the high-pressure air supplied to the head portion 20 to the outside. The high-pressure air supplied to the head portion 20 is discharged to the outside not only from the discharge duct but also from a gap formed between contact pieces 511b and 512b of the seal members 511 and 512 to be described later and the outer peripheral surface of the spindle 41.

The high-pressure air supplied from the supply duct is supplied to the cartridge 40 and ejected to the turbine blades 422 of the turbine rotor 42. When the turbine blades 422 of the turbine rotor 42 receive the high-pressure air, the turbine rotor 42 rotates. Accordingly, the spindle 41 and the cutting tool 30 rotate integrally with the turbine rotor 42.

The turbine rotor 42 is formed of a metal having a density of 4.0 [g/cm³] or more and a value of Young's modulus [GPa]/density [g/cm³] of 20 or more. When the turbine rotor 42 includes eight blades with a diameter of 10 mm × an axial length of 3.2 mm, an inertial moment of the turbine rotor 42 is 10.8 [g·mm²] if the turbine rotor 42 is formed of stainless steel. For reference, when a turbine rotor having the same shape is formed of aluminum, an inertial moment of the turbine rotor is 3.7 [g·mm²].

In this way, since the turbine rotor 42 is formed of the metal having the density of 4.0 [g/cm³] or more, the inertial moment of the turbine rotor 42 can be increased. Accordingly, when supply of the high-pressure air from the supply duct of the handpiece main body 10 to the head portion 20 is started, it is possible to prevent a sudden increase in a rotation speed of the turbine rotor 42 and to reduce a load applied to the first bearing 451 and the second bearing 452. When a cutting load is input to the turbine rotor 42 via the cutting tool 30 during rotation of the turbine rotor 42, it is possible to prevent a decrease in a cutting force of the cutting tool 30 due to stall of the turbine rotor 42.

Further, in the turbine rotor 42, the value of Young's modulus [GPa]/density [g/cm³] is 20 or more, and generally, when the same force acts on the turbine rotor having the same shape with different Young's moduli, the larger the Young's modulus, the less the deformation. When objects having the same shape with different densities are subjected to the same rotation, the higher the density, the larger a force acting on the object. Therefore, the larger the Young's modulus and the lower the density, the smaller an amount of deformation due to rotation. In the present embodiment, since the value of Young's modulus [GPa]/density [g/cm³] is 20 or more in the turbine rotor 42, and an amount of deformation of the turbine rotor 42 can be reduced even when the turbine rotor 42 is rotated at high speed. Therefore, even when the turbine rotor 42 is rotated at high speed, it is possible to prevent an increase in an inner diameter of the rotation shaft portion 421 and to prevent fixing between the turbine rotor 42 and the spindle 41 from loosing.

Further, in the air turbine handpiece 1, since a position of a center of gravity can be brought close to the head portion 20, a natural frequency of the turbine rotor 42 is increased when the high-pressure air is supplied from the supply duct of the handpiece main body 10 to the head portion 20 and the turbine rotor 42 rotates. Accordingly, when the high-pressure air is supplied from the supply duct of the handpiece main body 10 to the head portion 20 and the turbine rotor 42 rotates, it is possible to reduce unpleasant noise caused by the turbine rotor 42.

In the present embodiment, the turbine rotor 42 is formed of stainless steel. More specifically, in the present embodiment, the turbine rotor 42 is formed of SUS 303, which is austenitic stainless steel.

In this way, since the turbine rotor 42 is formed of stainless steel, which is a material that is easy to process, the turbine rotor 42 can be easily molded.

The turbine rotor 42 is formed of austenitic stainless steel, which is a material that is not easily corroded even after hot water cleaning, and thus has high corrosion resistance. Therefore, it is not necessary to perform a surface treatment on the turbine rotor 42 to improve corrosion resistance, such as coating with alumite.

Since the turbine rotor 42 is formed of the metal having the density of 4.0 g/ cm³ or more and the value of Young's modulus over density of 20 GPa/(g/cm³) or more, and the inertial moment is larger than that of related-art aluminum, an inertial force during rotation of the turbine rotor 42 also increases. Therefore, when the cutting load is input, the turbine rotor 42 is stopped through the cutting tool 30, and a load in a direction opposite to a rotation direction is applied to a joint portion 46 of the rotation portion 4. When a fixing force of the joint portion 46 between the turbine rotor 42 and the spindle 41 is weakened, the spindle 41 easily moves in the axial direction if a load acts in the axial direction, such as if the cutting load is input, which may cause a failure due to heat generation or poor attachment and detachment of the cutting tool.

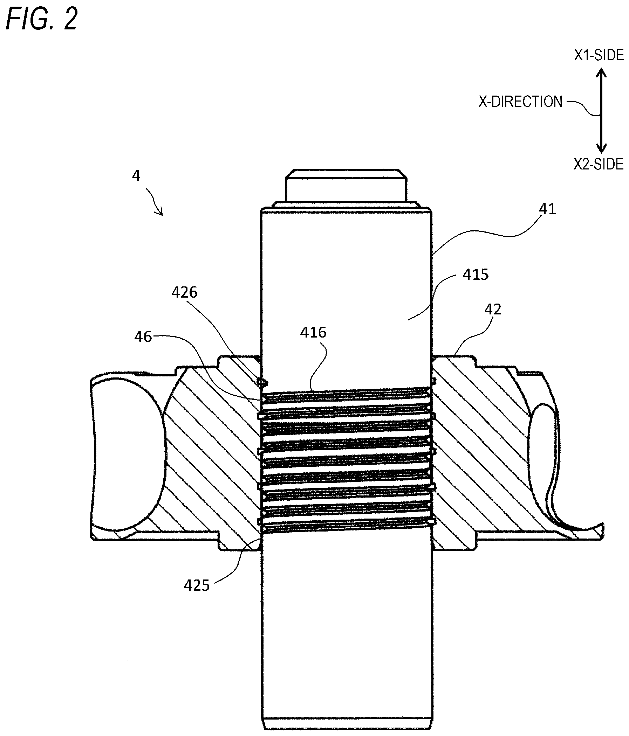

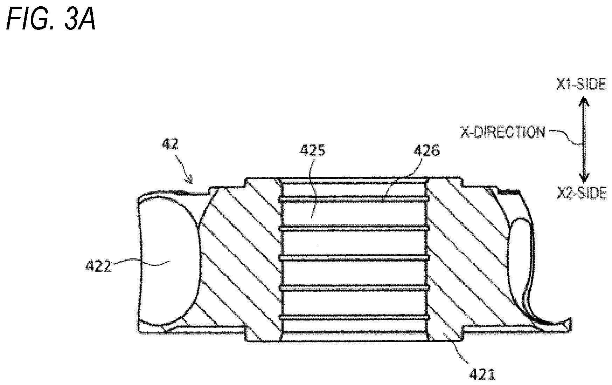

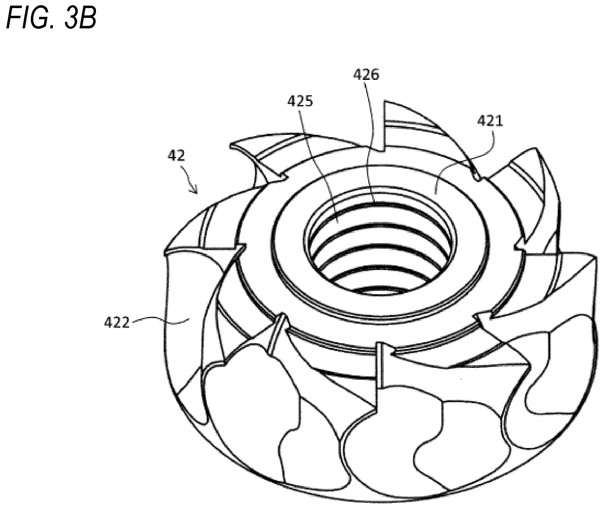

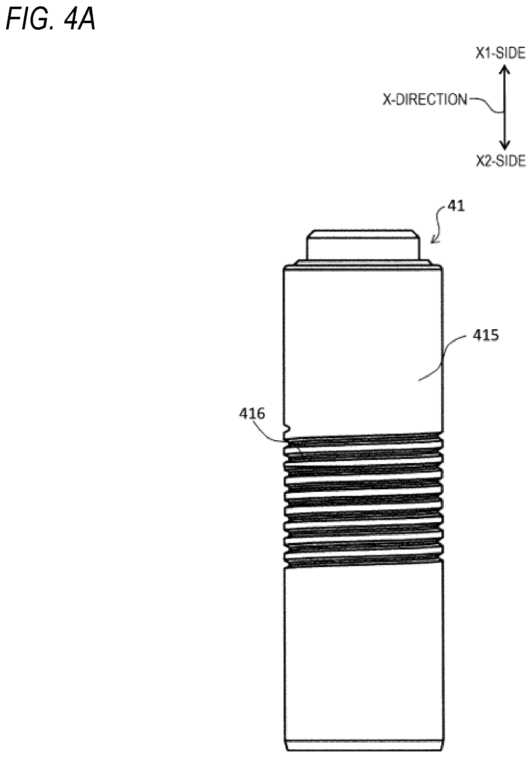

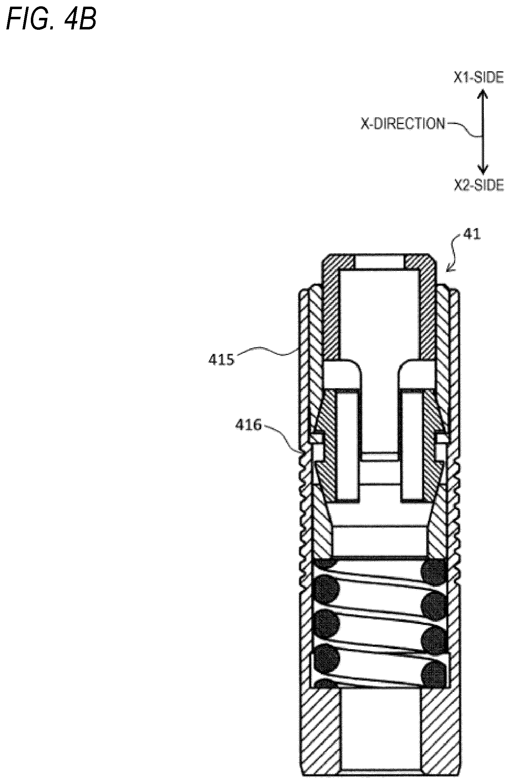

As shown in Fig. 2, the rotation portion 4 including the turbine rotor 42 and the spindle 41 has the joint portion 46 where an outer peripheral surface 415 of the spindle 41 is fixed to an inner peripheral surface 425 of the turbine rotor 42 by an adhesive. As shown in Figs. 3A and 3B, grooves 426 parallel to a peripheral direction are formed at regular intervals in the axial direction in the inner peripheral surface 425 of the turbine rotor 42, and as shown in Figs. 4A and 4B, grooves 416 that are spiral-shaped screw grooves are formed in the outer peripheral surface 415 of the spindle 41. Grooves are formed in both the inner peripheral surface 425 of the turbine rotor 42 and the outer peripheral surface 415 of the spindle 41 in the present embodiment, and grooves may be formed in at least one of the surfaces.

In this way, since the joint portion 46 has the grooves 426 in the inner peripheral surface 425 of the turbine rotor 42 and the grooves 416 in the outer peripheral surface 415 of the spindle 41, the adhesive acts on the grooves 426 and 416 and turns into resin, thereby increasing the fixing force of the joint portion 46. This can prevent the turbine rotor 42 from falling off the spindle 41.

In the present embodiment, the grooves 426 in the inner peripheral surface 425 of the turbine rotor 42 and the grooves 416 in the outer peripheral surface 415 of the spindle 41 intersect each other. The intersection of the grooves 426 in the inner peripheral surface 425 of the turbine rotor 42 and the grooves 416 in the outer peripheral surface 415 of the spindle 41 is not limited, for example, the grooves may intersect each other by being formed into screw grooves in opposite directions, rightward and leftward. Accordingly, the resin adhesive at the joint portion 46 acts as a wedge at an intersecting portion of the grooves 426 and the grooves 416, and it is possible to further increase the fixing force of the joint portion 46, thereby preventing the turbine rotor 42 from falling off the spindle 41.

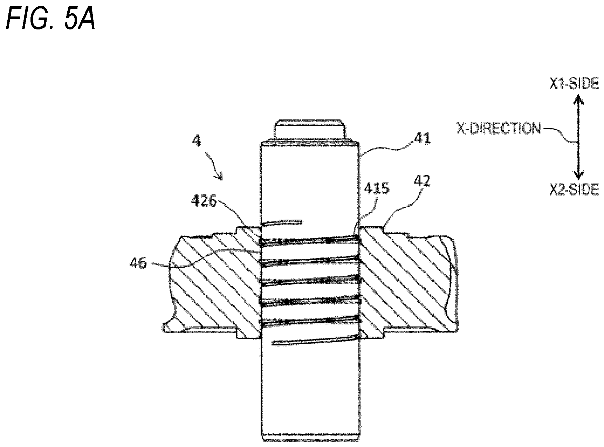

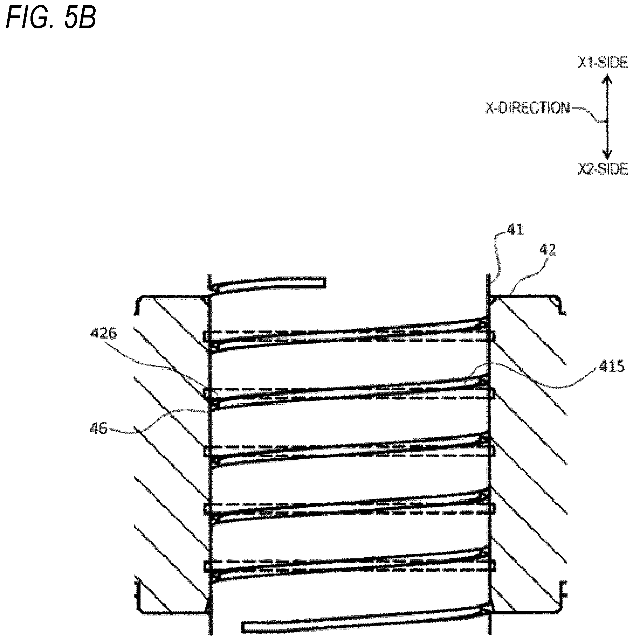

As shown in Figs. 5A and 5B, the grooves 426 parallel to the peripheral direction in the inner peripheral surface 425 of the turbine rotor 42 and the grooves 416 that are the spiral-shaped screw grooves in the outer peripheral surface 415 of the spindle 41 intersect each other at the joint portion 46 of the rotation portion 4. Since the grooves 426 in the inner peripheral surface 425 of the turbine rotor 42 are parallel to the peripheral direction, when an excessive load acts in the peripheral direction during rotation, adhesion of the grooves 426 in an outer peripheral direction is preferentially released, thereby preventing the resin adhesive from separating form the entire joint portion 46. The adhesion of the grooves 426 in the outer peripheral direction is released, thereby preventing the turbine rotor 42 from falling off the spindle 41 in the axial direction. The grooves 426 in the inner peripheral surface 425 of the turbine rotor 42 are grooves parallel to the peripheral direction in the present embodiment, but the present invention is not limited thereto, and the grooves 416 in the outer peripheral surface 415 of the spindle 41 may be grooves parallel to the peripheral direction.

The adhesive used in the present embodiment is an acrylic adhesive, preferably an acrylic resin-based anaerobic adhesive, and more preferably Loctite (registered trademark) 648.

The first bearing 451 includes an inner ring 451a and an outer ring 451b facing each other, and balls 451c located in a gap where the inner ring 451a and the outer ring 451b face each other.

A sandwiching portion 451d that extends radially inward and supports the seal member 511 from the X2-side is formed at an X1-side end portion of the outer ring 451b of the first bearing 451.

An extending portion 451e that extends radially outward and supports the O-ring 491 from the X2-side is formed at an X2-side end portion of the outer ring 451b of the first bearing 451.

An inner ring opening 451a1 is opened inside the inner ring 451a of the first bearing 451. The inner ring opening 451a1 is fixed to the outer peripheral surface of the spindle 41. Therefore, the inner ring 451a rotates together with the spindle 41.

A seal holding member 521 is attached to the outer ring 451b of the first bearing 451 on the X1-side. The seal holding member 521 is locked to an outer peripheral surface of the outer ring 451b of the first bearing 451. The seal holding member 521 includes a base portion 521a that faces an X1-side surface of the outer ring 451b of the first bearing 451, a sandwiching portion 521b that extends from the base portion 521a toward the X1-side and supports the seal member 511 from the X1-side, and a locking portion 521c that extends from the base portion 521a toward the X2-side, supports the O-ring 491 from the X1-side, and is locked to the outer peripheral surface of the outer ring 451b of the first bearing 451.

The seal member 511 is formed of an elastic material. The seal member 511 is formed of silicone rubber or fluororubber, for example. The seal member 511 may be formed of an elastic material other than silicone rubber and fluororubber. The seal member 511 has a ring shape surrounding the outer peripheral surface of the spindle 41 and having an open central portion. The seal member 511 includes an outer peripheral portion 511a that is the thickest and a contact piece 511b extending from an inner edge portion of the outer peripheral portion 511a toward a center of the ring. The contact piece 511b has a lower elastic modulus than the outer peripheral portion 511a and is a portion that is easy to be elastically deformed. In the seal member 511, a ring opening 511c is opened at a central portion of the ring further than the contact piece 511b.

The outer peripheral portion 511a of the seal member 511 is supported by the sandwiching portion 521b of the seal holding member 521 on the X1-side and supported by the sandwiching portion 451d of the first bearing 451 on the X2-side, and is thus sandwiched by the sandwiching portion 521b of the seal holding member 521 and the sandwiching portion 451d of the first bearing 451.

The contact piece 511b of the seal member 511 is disposed in a space surrounded by an inner peripheral surface of the sandwiching portion 521b of the seal holding member 521 and the outer peripheral surface of the spindle 41. When the turbine rotor 42 of the air turbine handpiece 1 is not operated, that is, when the high-pressure air is not supplied from the supply duct of the handpiece main body 10 to the head portion 20, the contact piece 511b is contact with the outer peripheral surface of the spindle 41.

A diameter of the ring opening 511c of the seal member 511 is smaller than a diameter of the outer peripheral surface of the spindle 41. Therefore, when the turbine rotor 42 of the air turbine handpiece 1 is not operated, that is, when the high-pressure air is not supplied from the supply duct of the handpiece main body 10 to the head portion 20, the contact piece 511b of the seal member 511 is curved toward the X1-side and is in contact with the outer peripheral surface of the spindle 41 with a contact pressure due to an elastic force. A position where the contact piece 511b is in contact with the outer peripheral surface of the spindle 41 is an opening edge of the ring opening 511c.

In this way, when the high-pressure air is not supplied from the supply duct of the handpiece main body 10 to the head portion 20 and the turbine rotor 42 does not rotate, the contact piece 511b of the seal member 511 abuts on the outer peripheral surface of the spindle 41 to close (seal) a gap between the head cap 22 and the spindle 41.

This may prevent foreign matter such as saliva and blood from entering the cartridge 40.

The second bearing 452 includes an inner ring 452a and an outer ring 452b facing each other, and balls 452c located in a gap where the inner ring 452a and the outer ring 452b face each other.

A sandwiching portion 452d that extends radially inward and supports the seal member 512 from the X1-side is formed at an X2-side end portion of the outer ring 452b of the second bearing 452.

An extending portion 452e that extends radially outward and supports the O-ring 492 from the X1-side is formed at an X1-side end portion of the outer ring 452b of the second bearing 452.

An inner ring opening 452a1 is opened inside the inner ring 452a of the second bearing 452. The inner ring opening 452a1 is fixed to the outer peripheral surface of the spindle 41. Therefore, the inner ring 452a rotates together with the spindle 41.

A seal holding member 522 is attached to the outer ring 452b of the second bearing 452 on the X2-side. The seal holding member 522 is locked to an outer peripheral surface of the outer ring 452b of the second bearing 452. The seal holding member 522 includes a base portion 522a that faces an X2-side surface of the outer ring 452b of the second bearing 452, a sandwiching portion 522b that extends from the base portion 522a toward the X2-side and supports the seal member 512 from the X2-side, and a locking portion 522c that extends from the base portion 522a toward the X1-side, supports the O-ring 492 from the X2-side, and is locked to the outer peripheral surface of the outer ring 452b of the second bearing 452.

The seal member 512 is formed of an elastic material. The seal member 512 is formed of silicone rubber or fluororubber, for example. The seal member 512 may be formed of an elastic material other than silicone rubber and fluororubber. The seal member 512 has a ring shape surrounding the outer peripheral surface of the spindle 41 and having an open central portion. The seal member 512 includes an outer peripheral portion 512a that is the thickest and a contact piece 512b extending from an inner edge portion of the outer peripheral portion 512a toward a center of the ring. The contact piece 512b has a lower elastic modulus than the outer peripheral portion 512a and is a portion that is easy to be elastically deformed. In the seal member 512, a ring opening 512c is opened at a central portion of the ring further than the contact piece 512b.

The outer peripheral portion 512a of the seal member 512 is supported by the sandwiching portion 452d of the second bearing 452 on the X1-side and supported by the sandwiching portion 522b of the seal holding member 522 on the X2-side, and is thus sandwiched by the sandwiching portion 452d of the second bearing 452 and the sandwiching portion 522b of the seal holding member 522.

The contact piece 512b of the seal member 512 is disposed in a space surrounded by an inner peripheral surface of the sandwiching portion 522b of the seal holding member 522 and the outer peripheral surface of the spindle 41. When the turbine rotor 42 of the air turbine handpiece 1 is not operated, that is, when the high-pressure air is not supplied from the supply duct of the handpiece main body 10 to the head portion 20, the contact piece 512b is in contact with the outer peripheral surface of the spindle 41.

A diameter of the ring opening 512c of the seal member 512 is smaller than a diameter of the outer peripheral surface of the spindle 41. Therefore, when the turbine rotor 42 of the air turbine handpiece 1 is not operated, that is, when the high-pressure air is not supplied from the supply duct of the handpiece main body 10 to the head portion 20, the contact piece 512b of the seal member 512 is curved toward the X2-side and is in contact with the outer peripheral surface of the spindle 41 with a contact pressure due to an elastic force. A position where the contact piece 512b is in contact with the outer peripheral surface of the spindle 41 is an opening edge of the ring opening 512c.

In this way, when the high-pressure air is not supplied from the supply duct of the handpiece main body 10 to the head portion 20 and the turbine rotor 42 does not rotate, the contact piece 512b of the seal member 512 abuts on the outer peripheral surface of the spindle 41 to close (seal) a gap between the main housing 21 and the spindle 41.

This can prevent foreign matter such as saliva and blood from entering the cartridge 40.

When the high-pressure air is supplied from the supply duct of the handpiece main body 10 to the head portion 20, an air pressure of the inner accommodation space 200 becomes high, and when the turbine rotor 42 rotates at a predetermined speed or more, the air from the inner accommodation space 200 leaks, through the first bearing 451, to a space where the contact piece 511b of the seal member 511 is disposed, and leaks, through the second bearing 452, to a space where the contact piece 512b of the seal member 512 is disposed.

Accordingly, the contact piece 511b of the seal member 511 is pushed radially outward from the spindle 41 by the air leaked into the space where the contact piece 511b of the seal member 511 is disposed, the contact piece 511b of the seal member 511 is separated from the outer peripheral surface of the spindle 41, and a gap is formed between the contact piece 511b of the seal member 511 and the outer peripheral surface of the spindle 41.

Similarly, the contact piece 512b of the seal member 512 is pushed radially outward from the spindle 41 by the air leaked into the space where the contact piece 512b of the seal member 512 is disposed, the contact piece 512b of the seal member 512 is separated from the outer peripheral surface of the spindle 41, and a gap is generated between the contact piece 512b of the seal member 512 and the outer peripheral surface of the spindle 41.

As described above, when the high-pressure air is supplied from the supply duct of the handpiece main body 10 to the head portion 20, the turbine blades 422 of the turbine rotor 42 receive the high-pressure air, whereby the turbine rotor 42 rotates, and the spindle 41 and the cutting tool 30 rotate integrally with the turbine rotor 42. When the high-pressure air is supplied from the supply duct of the handpiece main body 10 to the head portion 20, gaps are formed between the contact piece 511b of the seal member 511 and the outer peripheral surface of the spindle 41 and between the contact piece 512b of the seal member 512 and the outer peripheral surface of the spindle 41, and thus the spindle 41 has no contact resistance with the contact piece 511b of the seal member 511 and the contact piece 512b of the seal member 512, and can be easily rotated.

On the other hand, when supply of the high-pressure air from the supply duct of the handpiece main body 10 to the head portion 20 is stopped from a state in which the high-pressure air is supplied from the supply duct of the handpiece main body 10 to the head portion 20 and the turbine rotor 42, the spindle 41, and the cutting tool 30 are integrally rotated, a rotation speed of the turbine rotor 42, the spindle 41, and the cutting tool 30 is reduced, and the air leaked into the space where the contact piece 511b of the seal member 511 is disposed and the space where the contact piece 512b of the seal member 512 is disposed is reduced. When the rotation speed of the turbine rotor 42 becomes equal to or lower than a predetermined speed, the contact piece 511b of the seal member 511 and the contact piece 512b of the seal member 512, which are pushed radially outward from the spindle 41, return to a state of being in contact with the outer peripheral surface of the spindle 41. Rotation of the turbine rotor 42, the spindle 41, and the cutting tool 30 is braked by a contact resistance between the contact piece 511b of the seal member 511 and the outer peripheral surface of the spindle 41 and a contact resistance between the contact piece 512b of the seal member 512 and the outer peripheral surface of the spindle 41. In this way, the seal members 511 and 512 also function as a quick stop mechanism that brakes the rotation of the turbine rotor 42, the spindle 41, and the cutting tool 30 when the supply of the high-pressure air from the supply duct of the handpiece main body 10 to the head portion 20 is stopped. Accordingly, the seal members 511 and 512 can stop the rotation of the turbine rotor 42, the spindle 41, and the cutting tool 30 in a short time after the supply of the high-pressure air from the supply duct of the handpiece main body 10 to the head portion 20 is stopped.

Since the air turbine handpiece 1 includes the seal members 511 and 512 that also function as the quick stop mechanism that brakes the rotation of the turbine rotor 42, the spindle 41, and the cutting tool 30 when the supply of the high-pressure air from the supply duct of the handpiece main body 10 to the head portion 20 is stopped, even when a material having a large inertial moment is used for the turbine rotor 42, the rotation of the turbine rotor 42, the spindle 41, and the cutting tool 30 can be stopped in a short time after the supply of the high-pressure air from the supply duct of the handpiece main body 10 to the head portion 20 is stopped.

In the present embodiment, since the seal members 511 and 512 are provided on both the X1-side of the first bearing 451 and the X2-side of the second bearing 452, even when the material having the large inertial moment is used for the turbine rotor 42, it is possible to further increase a braking force for braking the rotation of the turbine rotor 42, the spindle 41, and the cutting tool 30 when the supply of the high-pressure air from the supply duct of the handpiece main body 10 to the head portion 20 is stopped.

Since the seal members 511 and 512 function as the quick stop mechanism that brakes the rotation of the turbine rotor 42, the spindle 41, and the cutting tool 30, the quick stop mechanism can be provided without increasing the number of components.

The air turbine handpiece 1 is used after a lubricant is injected into the head portion 20 before use. Therefore, the air turbine handpiece 1 is used in a state in which the lubricant is coated on each component inside the head portion 20. For example, first, the lubricant is sufficiently injected into the head portion 20 from the supply duct of the handpiece main body 10 to remove dirt attached to each component inside the head portion 20, and the lubricant is coated on each component inside the head portion 20. Thereafter, the air turbine handpiece 1 is used after removing the excess lubricant.

Accordingly, it is possible to prevent a defect in each component inside the head portion 20 due to adhesion of dirt, and to prevent wear of the first bearing 451 and the second bearing 452. Since the outer peripheral surface 415 of the spindle 41 and the seal members 511 and 512 are also coated with the lubricant when using the air turbine handpiece 1, during using of the air turbine handpiece, when the supply of the high-pressure air from the supply duct of the handpiece main body 10 to the head portion 20 is stopped from the state in which the turbine rotor 42, the spindle 41, and the cutting tool 30 are integrally rotated, and the rotation of the turbine rotor 42, the spindle 41, and the cutting tool 30 is braked due to the contact resistances between the contact pieces 511b and 512b of the seal members 511 and 512 and the outer peripheral surface of the spindle 41, it is possible to prevent wear of the contact pieces 511b and 512b of the seal members 511 and 512.

As described above, since the quick stop mechanism including the seal members is provided, it is possible to stop, in a short time, long time inertial rotation that occurs when the material having the large inertial moment is used for the turbine rotor. On the other hand, when the seal members 511 and 512 of the quick stop mechanism come into contact with the outer peripheral surface of the spindle 41, an additional load is applied to the joint portion 46 of the rotation portion 4 due to sudden braking, but the joint portion 46 has a high fixing force due to the grooves 426 and/or the grooves 416, thereby preventing the turbine rotor 42 from falling off the spindle 41.

### Second Embodiment

Next, a second embodiment of the present invention will be described. In the following description, the same components as those of the air turbine handpiece 1 according to the first embodiment are denoted by the same reference numerals, and the description thereof will be omitted or simplified. Hereinafter, differences from the air turbine handpiece 1 according to the first embodiment will be described in detail.

The second embodiment is different from the first embodiment in that a material of the turbine rotor 42 of the air turbine handpiece 1 is not limited.

Accordingly, even when the turbine rotor 42 is formed of related-art aluminum, an adhesive acts on the grooves 426 in the inner peripheral surface 425 of the turbine rotor 42 and the grooves 416 in the outer peripheral surface 415 of the spindle 41 and turns into resin, thereby increasing a fixing force of the joint portion 46.

Although the embodiments of the present invention have been described above with reference to the accompanying drawings, it is needless to say that the present invention is not limited to these embodiments. It is apparent to those skilled in the art that various changes or modifications can be conceived within the scope described in the claims, and it is understood that the changes or modifications naturally fall within the technical scope of the present invention. In addition, the components in the above embodiments may be combined as desired without departing from the scope of the invention.

For example, instead of stainless steel, metal such as titanium may be used for the turbine rotor 42 according to the first embodiment.

For example, in the first embodiment and the second embodiment, a plurality of seal members 511 and 512 may be provided on the X1-side of the first bearing 451 and/or a plurality of seal members 511 and 512 may be provided on the X2-side of the second bearing 452. By increasing the number of the seal members 511 and 512, even when a material having a large inertial moment is used for the turbine rotor 42, it is possible to further increase a braking force for braking rotation of the turbine rotor 42, the spindle 41, and the cutting tool 30 when supply of high-pressure air from the supply duct of the handpiece main body 10 to the head portion 20 is stopped.

For example, the spindle 41, the turbine rotor 42, the chuck mechanism portion 44, the first bearing 451 and the second bearing 452, and the seal members 511 and 512 are modularized as the cartridge 40 that is attachable to and detachable from the head portion 20 in the first embodiment and the second embodiment, and the spindle 41, the turbine rotor 42, the chuck mechanism portion 44, the first bearing 451, the second bearing 452, and the seal members 511 and 512 may not be modularized.

For example, the seal members 511 and 512 are fixed to the seal holding members 521 and 522 of the cartridge 40 in the first embodiment and the second embodiment, and the seal members 511 and 512 may be provided on the first bearing 451 and/or the second bearing 452.

For example, the turbine rotor 42 is formed of the material having the large inertial moment in the first embodiment, and the turbine rotor 42 may include a pair of weights provided in the X-direction on an X1-side surface and an X2-side surface of the turbine rotor 42. The weight is formed of a material having a higher density than the material of the turbine rotor 42. Each of the pair of weights has a ring shape centered on a rotation axis of the turbine rotor 42 and is fitted to the outer peripheral surface of the spindle 41, and thus can be retrofitted.

For example, the grooves 426 parallel to the peripheral direction are formed at regular intervals in the axial direction in the inner peripheral surface 425 of the turbine rotor 42, and the grooves 416 that are the spiral-shaped screw grooves are formed in the outer peripheral surface 415 of the spindle 41 in the first embodiment and the second embodiment, and the shapes, sizes, angles, depths, intervals, and the like of the grooves 426 and 416 are not limited as long as the adhesive acts on the grooves 426 and 416 and turns into resin, thereby increasing the fixing force of the joint portion 46.

## Claims

1. A cartridge detachably attachable to a medical cutting device, wherein the cartridge comprises:
a rotation portion including a turbine rotor (42) and a spindle (41),
wherein the turbine rotor is formed of a metal having a density of 4.0 g/cm³ or more and a value of Young's modulus over density of 20 GPa/(g/cm³) or more,
wherein the spindle has a hollow and substantially cylindrical shape and a cutting tool is to be inserted into a hollow interior, wherein the spindle further has an outer peripheral surface (415) that is rotatably supported by a first bearing (451) and a second bearing (452) and a chuck mechanism portion (44) supported on an inner peripheral surface of the spindle,
wherein the rotation portion has a joint portion where an inner peripheral surface of the turbine rotor is fixed to the outer peripheral surface of the spindle by an adhesive, and
wherein the joint portion has grooves in at least one of the inner peripheral surface of the turbine rotor and the outer peripheral surface of the spindle.

2. The cartridge according to claim 1,
wherein the joint portion has the grooves (416, 426) in the inner peripheral surface of the turbine rotor and the outer peripheral surface of the spindle.

3. The cartridge according to claim 2,
wherein the grooves (416, 426) in the inner peripheral surface of the turbine rotor and the outer peripheral surface of the spindle intersect each other at the joint portion.

4. The cartridge according to claim 3,
wherein at least one of the grooves in the inner peripheral surface of the turbine rotor and the outer peripheral surface of the spindle are parallel to a peripheral direction at the joint portion.

5. The cartridge according to claim 1, further comprising:
a quick stop mechanism including a seal member (511, 512) that surrounds the outer peripheral surface of the spindle, abuts on the outer peripheral surface of the spindle when the turbine rotor does not rotate, and is separated from the outer peripheral surface of the spindle when the turbine rotor rotates at a predetermined speed or more.

6. A medical cutting device comprising:
a handpiece main body (10);
a head portion (20) provided at a tip end portion of the handpiece main body (10); and
the cartridge (40) according to any one of the preceding claims, the cartridge being configured to be detachably attachable to the head portion (20).

## Patentansprüche

1. Kartusche, die lösbar und ansteckbar an einer medizinischen Schneidvorrichtung angebracht ist, wobei die Kartusche umfasst:
einen Drehabschnitt, der einen Turbinenläufer (42) und eine Spindel (41) enthält,
wobei der Turbinenläufer aus einem Metall gebildet ist, das eine Dichte von 4,0 g/cm³ oder mehr aufweist, und das einen Young-Modulwert über der Dichte von 20 GPa/(g/cm³) oder mehr aufweist,
wobei die Spindel einen Hohlraum und eine im Wesentlichen zylindrische Form aufweist, und wobei das Schneidwerkzeug in einen inneren Hohlraum eingesetzt werden soll, wobei die Spindel ferner eine äußere Randoberfläche (415), die drehbar von einem ersteh Lager (451) und einem zweiten Lager (452) getragen wird, und einen Spannmechanismusabschnitt (44) aufweist, der von einer inneren Randoberfläche der Spindel getragen wird,
wobei der Drehabschnitt einen gemeinsamen Abschnitt aufweist, an dem eine innere Randoberfläche des Turbinenläufers an der äußeren Randoberfläche der Spindel durch ein Haftmittel befestigt ist, und
wobei der gemeinsame Abschnitt Einschnitte an der inneren Randoberflächen des Turbinenläufers und/oder an der äußeren Randoberfläche der Spindel aufweist.

2. Kartusche nach Anspruch 1,
wobei der gemeinsame Abschnitt die Einschnitte (416, 426) in der inneren Randoberfläche des Turbinenläufers und in der äußeren Randoberfläche der Spindel aufweist.

3. Kartusche nach Anspruch 2,
wobei sich die Einschnitte (416, 426) in der inneren Randoberfläche des Turbinenläufers und in der äußeren Randoberfläche der Spindel gegenseitig an dem gemeinsamen Abschnitt schneiden.

4. Kartusche nach Anspruch 3,
wobei die Einschnitte in der inneren Randoberfläche des Turbinenläufers und/oder in der äußeren Randoberfläche der Spindel parallel zu einer Randrichtung des gemeinsamen Abschnitts sind.

5. Kartusche nach Anspruch 1, die ferner umfasst:
einen schnellen Stopp-Mechanismus, der ein Dichtungselement (511, 512) enthält, das die äußere Randoberfläche der Spindel umgibt, an die äußere Randoberfläche der Spindel angrenzt, wenn sich der Turbinenläufer nicht dreht, und das von der äußeren Randoberfläche der Spindel getrennt ist, wenn sich der Turbinenläufer mit einer vorgegebenen Geschwindigkeit oder mehr dreht

6. Medizinische Schneidvorrichtung, die umfasst:
einen Handstück-Hauptkörper (10);
einen Handabschnitt (20), der an einer Spitze eines Endabschnitts des Handstück-Hauptkörpers (10) bereitgestellt ist; und
die Kartusche (40) nach einem der vorhergehenden Ansprüche, wobei die Kartusche konfiguriert ist, um lösbar und ansteckbar an dem Handabschnitt (20) zu sein.

## Revendications

1. Cartouche pouvant être fixée de manière séparable à un dispositif de coupe médical, dans laquelle la cartouche comprend :
une partie rotative incluant un rotor de turbine (42) et une broche (41),
dans laquelle le rotor de turbine est formé d'un métal ayant une densité de 4,0 g/cm³ ou plus et une valeur de module de Young sur la densité de 20 Gpa/(g/cm³) ou plus,
dans laquelle la broche a une forme creuse et essentiellement cylindrique et un outil de coupe est destiné à être inséré dans un intérieur creux, dans laquelle la broche comprend en outre une surface périphérique externe (415) qui est supportée en rotation par un premier palier (451) et un second palier (452) et une partie de mécanisme de mandrin (44) supportée sur une surface périphérique interne de la broche,
dans laquelle la partie rotative présente une partie de jointure où une surface périphérique interne du rotor de turbine est fixée à la surface périphérique externe de la broche par un adhésif, et
dans laquelle la partie de jointure présente des rainures dans au moins une de la surface périphérique interne du rotor de turbine et de la surface périphérique externe de la broche.

2. Cartouche selon la revendication 1,
dans laquelle la partie de jointure présente les rainures (416, 426) dans la surface périphérique interne du rotor de turbine et la surface périphérique externe de la broche.

3. Cartouche selon la revendication 2,
dans laquelle les rainures (416, 426) dans la surface périphérique interne du rotor de turbine et la surface périphérique externe de la broche se coupent entre elles à la partie de jointure.

4. Cartouche selon la revendication 3,
dans laquelle au moins une des rainures dans la surface périphérique interne du rotor de turbine et la surface périphérique externe de la broche sont parallèles à une direction périphérique à la partie de jointure.

5. Cartouche selon la revendication 1, comprenant en outre :
un mécanisme d'arrêt rapide incluant un membre d'étanchéité (511, 512) qui entoure la surface périphérique externe de la broche, affleure sur la surface périphérique externe de la broche lorsque le rotor de turbine ne tourne pas, et est séparé de la surface périphérique externe de la broche lorsque le rotor de turbine tourne à une vitesse prédéterminée ou supérieure.

6. Dispositif de coupe médical comprenant :
un corps de pièce à main (10) ;
une partie de tête (20) prévue à une partie d'extrémité en pointe du corps de pièce à main (10) ;
et
la cartouche (40) selon l'une quelconque des revendications précédentes, la cartouche étant configurée pour être fixée de manière séparable à la partie de tête (20).
